# EUROPEAN PATENT APPLICATION

(11) **EP 2 105 736 A1**
(43) Date of publication of application: **30.09.2009**
(21) Application number: 08006033.8
(22) Date of filing: 28.03.2008
(51) Int. Cl.: G01N 27/447

(54) **Analysis of DNA by means of cappillary electrophoresis**

(71) Applicant: Novartis AG, 4056 Basel (CH)
(72) Inventor: Kost, Holger, Dr., 35713 Eschenburg (DE)
(74) Representative: UEXKÜLL & STOLBERG

(57) **Abstract**

The present invention relates to a method for detecting nucleic acids, wherein a sample to be analyzed for the presence of nucleic acids is separated by capillary electrophoresis. The conditions of sample injection and separation allow for an extremely high sensitivity of the method, which can be applied, e.g. for quality control purposes in the determination or the presence, quantity and/or size of genomic DNA contaminants in samples comprising proteins for therapy or vaccination.

## Description

The present invention relates to a method for detecting nucleic acids, wherein a sample to be analyzed for the presence of nucleic acids is separated by capillary electrophoresis. The conditions of sample injection and separation allow for an extremely high sensitivity of the method, which can be applied, e.g. for quality control purposes in the determination or the presence and/or size of genomic DNA contaminants in samples intended for therapy or vaccination, in particular against influenza.

Influenza is a disease caused by a virus from the group of orthomyxoviruses. It is mainly type A, rarely type B and practically never type C which is responsible for the disease.

The best prevention measure against influenza is vaccination, which is available against influenza type A and B. Vaccines against influenza have been known since 1952. The conventional approach of propagation of virus in eggs requires at least six months for production of a vaccine. The use of cell culture is an alternative approach that has several advantages over use of eggs.

For products prepared in cell culture, one parameter assessed by regulatory authorities is the content of residual host cell DNA, due to its transforming potential. One possible way of minimalizing risks associated with host cell DNA is to reduce the amount of DNA present in the vaccine. Alternatively, it can be shown that nucleic acids remaining in the final product have lost their oncogenic potential.

In relation to MDCK cells (Madin-Darby canine kidney cells), the genome of *Canis familiaris* has been completely sequenced in 2004; and it is available on the internet. In studies of Novartis Vaccines, it was shown that only 13 of the about 25000 genes with a specific function have a length of less than 500 bp. None of these 13 genes was found to have any oncogenic potential (Internal data obtained from database search).

This finding revealed a need to develop a highly sensitive method for the analysis of samples potentially comprising nucleic acids, such as vaccines, for the presence and size distribution of the nucleic acids.

To assess the amount and size distribution of nucleic acids during the process of vaccine preparation, samples from different process stages can be analysed. This leads to the additional difficulty that these in-process samples differ from each other in their chemical composition. There are huge differences in the concentration of DNA contained, which can be up to three orders of magnitude. Thus, development of a method for analysis is a challenging task.

Quantification of absolute concentrations of nucleic acids can be achieved with several methods. For example, photometric detection of the optical density at 280 nm and 260 nm allows conclusions both about the nucleic acid concentration and protein content of a sample. Tests based on fluorescent dyes are more sensitive. For example, PicoGreen^{®} has a detection limit of less than about 312 pg/ml dsDNA. This test is however very sensitive to impurities in the sample, and results have a high degree of variation. The Threshold System^{®} test makes it possible to quantify DNA in a concentration between 6,2-400 pg/ml. However, these tests do not provide any qualitative information.

For qualitative analysis of nucleic acids such as genomic DNA, classical agarose slab gel electrophoresis is most frequently used. Accordingly, as described in detail in the examples below, it was first attempted to analyse samples from the production of the influenza vaccine by means of agarose gel electrophoresis. While in the samples from the initial purification steps, DNA could be detected by this method, the concentration in the samples from latter steps and the final product was too low to allow analysis. Similarly, it was found that polyacrylamide gel electrophoresis was not suitable for analysis of the samples.

It is known that capillary gel electrophoresis can be used for the analysis of a broad range of substances, such as oligonucleotides or DNA [C. Heller, Electrophoresis 629, Issue 2 (2001); Menzinger et al., Analysis of agrochemicals by capillary electrophoresis, J. Chromatogr. A 891 (2000), 45; Mitchelson et al., Capillary electrophoresis of nucleic acids, Vol II, Practical applications of capillary electrophoresis, Humana Press Totowa, New Jersey 2001]. This method has a high efficiency and sensitivity and can be quickly performed.

Surprisingly, the present inventors found that capillary gel electrophoresis can be adapted to provide a highly sensitive and reliable test for the analysis of nucleic acids, in particular DNA, during various steps of vaccine production. The resultant method satisfies the needs in the prior art and solves the problem underlying the present invention.

The present invention provides a method for analysing the presence and/or size distribution of nucleic acids in a sample, wherein the sample is separated by capillary gel electrophoresis, comprising
a) a hydrodynamic pre-injection of the capillary with water at about 7 to about 35 kPa for 2 to 10 s, preferably at about 20 or 21 kPa for about 5 s,
b) electrokinetic injection of the sample at 5-15 kV for 10-60 s, preferably at about 10 kV for about 30 s,
c) a hydrodynamic post-injection with water at about 3 to 14 kPa for 2-10 s, preferably at about 7 kPa for about 5 s,
d) separation at 200-275 V/cm, preferably at 225 to 250 V/cm,
e) detection of nucleic acids.

In the context of the invention, hydrodynamic injection can be achieved by applying pressure, such as hydrostatic pressure, at the inlet of the capillary, or by generating a vacuum or negative pressure at the outlet of the capillary. Normally, the sample is loaded by generating a pressure difference between the sample vial and the end of the capillary, wherein the pressure is raised at the sample vial. For injection by hydrostatic pressure (influence of gravitation), the sample vial at the inlet of the capillary is raised to a certain height. The difference between the level of buffer and the level of sample, as well as the density of the sample, influences loading. In practice, a product of pressure and injection time is used for the calculation of the injected amount of sample with a slow rise and fall of the pressure.

Electrokinetic injection is based on an electrophoretic and an electroosmotic movement generated by an electrical field in the capillary. When the inlet of the capillary extends into the sample vial, and voltage is applied for some seconds, the charged components of the sample migrate into the vial. The concentration of injected sample components can be varied by changing injection time or voltage. Accordingly, in the context of the invention, these can be varied to achieve injection of a suitable amount of sample. The injected amount of sample is also dependent on the electroosmotic flux in the capillary and mobility of the sample components. Nucleic acids within the capillary can be detected by any suitable method that reaches the sensitivity required by the desired application. Fluorescence detection, e.g., allows for excellent sensitivity and selectivity with a low detection limit (i.e., high sensitivity), especially if laser light is used for excitation (laser induced fluorescence, LIF). LIF is about 2 to 100 times more sensitive than UV detection and provides for a very high linearity of the signal. In extreme cases, sensitivity may extend to detection of single molecules. Thus, in the methods of the invention, detection is preferably by laser-induced fluorescence.

For detection of DNA, LIF detection can be used in different ways. The first method is based on the natural fluorescence of native DNA in the lower UV range. It allows analysis of DNA in its natural environment. For example, pulsed KrF 248 nm laser or UV laser with 275 nm or other lasers with wavelengths in this or a similar range are used as sources for excitation.
The second variant employs indirect fluorescence. A fluorescent capillary zone electrophoresis system is excited with a laser (e.g., 325 nm He-Cd laser) during separation of the nucleotides or the DNA. A further method is usually used for DNA sequencing and requires a direct covalent labelling of the analyte with suitable fluorophors.

In the most widely used method, intercalating dyes are employed, which integrate into nucleic acids and change the length, conformation and charge of the molecule. The complex of dye and nucleic acid is strongly fluorescent under light of the excitation wavelength, while the free dye is not. For this method, a 488 nm Ar ion laser is most suitable. Ethidiumbromide is the most common intercalating dye. In addition, derivatives thereof, often as monomeric or dimeric intercalators, are available. For example, the dye thiazol orange (TO) allows a very high sensitivity of detection. The dyes POPO-3, YOYO-3 and YOYO-1 are even more sensitive. The preferred dye is EnhanCE dye (Beckman Coulter, Fullerton, USA). Further useful dyes are disclosed in WO 03/089586.

In the context of the invention, it is preferred that the separation buffer for capillary gel electrophoreses comprises a dye suitable for detecting the nucleic acid, preferably an intercalating dye, most preferably EnhanCE dye. The nucleic acids are thus stained on column.

To improve reliability and robustness of the method, it is recommended that a washing step is carried out after steps a, b and/or c by contacting the ends of the capillary with water.

It has been found that the reliability of the method for routine applications can be further enhanced if an internal standard for allocation of relative mobility values is injected between step a and b, preferably at 7-34 kPa for 1-10 s, most preferably at about 20 kPa or 21 kPa for about 5 s. However, as discussed below, analysis of the data can also be carried out with reference to time instead of mobility. The internal standard (ISTD) should be selected to minimize the risk of interference with detection of a nucleic acid from the sample. In the context of analysis of samples from influenza vaccine production, the ISTD may be, e.g., a ss or ds DNA fragment, in particular a ds DNS fragment with a length of 20 to 300 bp, preferably about 200 bp. The ISTD may also be a ssDNA of less than 50 bp, e.g., a 23 bp ssDNA primer.

Aditionally, a solution of a detectable fluorescent dye such as fluorescein, e.g., fluorescein diluted 1:10 in water, may be applied after injection of the water plug and before sample injection, i.e., between steps (a) and (b). For example, the fluorescein solution is injected hydrodynamically at about 7 to 34 kPa for 2 to 10 s, preferably at about 21 kPa for about 5 s. This peak is detected before the smallest standard peak and serves two ends: firstly, it is a mobility marker for the standard, and secondly, it serves as a control of the laser.

The ISTD and the solution of the detectable fluorescent dye may be injected together or separately in either order.

In the context of analysis of samples from influenza vaccine production, the method of the invention is applied to analysis of DNA, preferably genomic DNA from a host cell and/or degradation products thereof. However, the method can also be advantageously applied for detection of DNA in other samples, e.g., analysis of DNA of defined length, gene therapy vectors,

RNA or ssDNA.

In its most preferred embodiment, in the method of the invention, the sample to be analyzed for the presence and/or size distribution of DNA is separated by capillary gel electrophoresis, comprising
a) a hydrodynamic pre-injection of the capillary with water at about 21 kPa for 5 s,
b) electrokinetic injection of the sample at 10 kV for 90 s,
c) a hydrodynamic post-injection with water at about 7 kPa for 5 s,
d) separation at 250 V/cm,
e) detection of nucleic acids by laser-induced fluorescence,
   wherein the separation buffer comprises an intercalating dye such as EnhanCE dye, wherein an internal standard and a fluorescein solution are injected between step a and b at about 21 kPa for 5 s, together or separately, and wherein a washing step is carried out after the steps of injection of the internal standard, after step b and/or after step c.

The present invention provides a method for qualitative analysis of nucleic acids with a sensitivity of at least 100 pg/ml, at least 50 pg/ml, at least 10 pg/ml, at least 9 pg/ml, at least 5 pg/ml, or at least 1 pg/ml, for a DNA fragment of 72 bp.

The most preferred parameters for sample loading and separation of the sample, which are further described below, were determined for a fused silica capillary with a neutral inner coating, preferably a polacrylamide coating. It is preferred that the capillary has an inner diameter of 75 µm to 125 µm, preferably of 100 µm.

Most preferred is the capillary available with the eCAP dsDNA kit from Beckman Coulter (eCap DNA Capillary, 100 µm I.D. 477477). The parameters given can be transferred to other neutrally coated capillaries. For other capillaries, the settings might need to be slightly adapted, in particular, within the range given, to achieve optimal results.

To improve the stability of the capillary, an outer polyimid coating is usually present. As described below, it is preferred that up to 2 mm at both ends of the capillary are not coated on the outer side to improve reliability of the method. Additionally, the outer coating needs to be removed at the site of detection.

Although a longer capillary can be used, e.g, 60 cm with 50 cm to the detector, it has surprisingly been found that the quality of results is not affected if the distance to the detector is, e.g., 40 cm (e.g., 50 cm total length). The decrease in the capillary length results in sorter separation times. Depending on the length to the detector, separation can be carried out for up to 40 min or longer. However, it is preferred that the separation is carried out for 20 to 35 min, preferably 23 to 30 min.

The temperature of the system during separation is 17-30°C, preferably 18 to 25°C. Best results have been found with 20°C.

To eliminate potential problems with microbubbles forming in the capillary, a pressure of about 14-69 kPa, preferably about 34 kPa, is applied to the capillary during separation.

The system for capillary gel electrophoresis that is most preferably used in the method of the invention is a ProteomeLab PA 800 Protein Characterization System (Beckman Coulter).

The preferred separation buffer is a Tris-Borate buffer with a pH of 8 to 9.5, preferably a pH of 8.9, which is a non crosslinked, physical gel with low viscosity, e.g., available in the eCAP dsDNA kit from Beckman Coulter.

In a preferred embodiment of the invention, the sample to be analysed is a pharmaceutical composition for therapy or vaccination. It is preferred that the sample is analyzed for the presence of genomic DNA and/or degradation products thereof. It is most preferred that the sample is an in-process sample or the final product from the process of vaccine preparation, in particular a vaccine against influenza. For example, these can be any of the B1 to B8 samples defined below or a sample from the trivalent bulk of the vaccine preparation.

Preferably the vaccine is an influenza vaccine prepared from cell culture, e.g., from MDCK, PER.C6, Vero cells. The vaccine may be a whole virion, split virion or purified surface glycoproteins.

For some samples, in particular those comprising significant amounts of proteins or high concentrations of salts, as described below in detail, significantly better results are obtained if, before loading for capillary electrophoresis, the sample has been pretreated with a method comprising steps wherein
i) the sample is digested with Proteinase K, preferably in the presence of SDS,
ii) nucleic acids are extracted, and
iii) optionally, nucleic acids are concentrated by centrifugation over a membrane with a cut-off size of 10 kDa.

The conditions for carrying out these steps are well known in the state of the art and/or described below.

In one aspect, the present invention provides a method for determining the size of a nucleic acid, comprising carrying out the method described above. The size of the nucleic acid is determined by comparison with an internal or external size standard. Preferably, in the context of analysis of samples from influenza vaccine production, external size standards are used, which should be run before and after each series of samples.

The present invention also provides a method for determining the size distribution of nucleic acids in a sample, comprising carrying out the method described above, wherein the size distribution of the nucleic acids is determined by comparison with the internal or external size standard.

Preferably, the signal detected in step e of the method is transformed into a curve showing intensity versus time or mobility, which is compared to time or mobility of the size standard(s) for assigning a size to a nucleic acid.

If the size distribution of nucleic acids in a sample is of interest, it may be analysed to determine the percentage of nucleic acids in the size range of interest. To this end, the area under the curve is calculated for a size range of interest (i.e., between the end points of the size range of interest, e.g., 0-500 bp) and compared with the total area under the curve to obtain the percentage of nucleic acids in the size range of interest.

Due to its sensitivity, reliability and robustness, the method of the invention can be advantageously used for quality control of a sample comprising proteins or nucleic acids for therapy or vaccination. The method is thus preferably used after or in parallel to the preparation of a vaccine, preferably a vaccine against influenza.

The present invention also provides a method of analysing the presence and/or size distribution of nucleic acids in a sample from various steps of a vaccine preparation, e.g., of an influenza vaccine, comprising separating the sample by capillary gel electrophoresis and detecting nucleic acids by laser induced fluorescence. Preferably, the nucleic acids are genomic DNA and/or degradation products thereof. The sample can be an in-process sample from the preparation process or the final product, e.g., a monovalent bulk or trivalent bulk from preparation of an influenza vaccine. The preferred methods of pretreating and/or loading the sample and carrying out the analysis can advantageously be employed to achieve optimal sensitivity and reliability of the method.

The experiments leading to the invention and preferred embodiments thereof are described in the examples below, which are intended to illustrate the invention. It will easily be recognized by the skilled person that modifications can be made and that some optimization steps can advantageously be used with or without employing other measures.

All cited publications are herewith fully incorporated herein.

### Examples

### 1. Samples

For the preparation of the vaccine, MDCK cells (Madin Darby Canine Kidney) can be employed following protocols known in the state of the art, e.g., for preparation of the product Optaflu admitted by the EMEA. Preferably, the suspension cell line MDCK-CDM (Novartis Vaccines) is used.

Briefly, for preparation of the influenza subunit vaccine, influenza viruses are cultivated in an MDCK-CDM suspension culture and purified by a process comprising several steps.

After the virus harvest has been cleared by centrifugation, a filtration (0.45 µm) and a cation exchange chromatography is carried out. The bound virus is eluted from the column using a NaCl solution and the virus is then concentrated. The virus is inactivated with betapropriolacton (BPL), which also heavily damages any MDCK DNA that is still present.

The surface antigens for preparation of the subunit vaccine, hemagglutinin and neuraminidase, are solubilized by CTAB (Cetyltrimethylammoniumbromide), and the virus cores are eliminated by ultracentrifugation. CTAB is removed. Subsequently, a filtration over a membrane of 22 µm is carried out. These steps are followed by anion exchange chromatography and a dia-ultrafiltration. The purification is finished with filtration. Thereby, the antigen concentrate, also called monovalent bulk or monobulk, is obtained. The microfiltrated monobulk is sent to the mixing plant for formulation of the vaccine.

The process can also be seen from the following diagram,
wherein the volumes in the respective steps are given and samples taken after a certain step are indicated by B1-B8) :
- Cultivation of cells in spinner bottles and
- Cultivation of virus
- Separation and filtration (B1)
- Cleared virus harvest
- cation exchange chromatography (B2)
- First concentration/diafiltration (B3)
- Inactivation/hydrolysis (B4)
- CTAB-treatment/ultracentrifugation
- First sterile filtration (0,2 µm) (B5)
- adsorber treatment (30 1)
- Second sterile filtration (0,2 µm) (B6)
- Anion exchange chromatography (B7)
- Second concentration/diafiltration (10 1)
- Sterile filtration (0,2 µm) (B8)
- Antigen concentrate / monovalent bulk (10 1)

After the monovalent bulk is obtained, a trivalent bulk comprising antigens from three different virus strains (usually two A strains and one B strain) is generated. Current vaccines are mostly trivalent vaccines. If all specifications and safety requirements are complied with, the vaccine is ready for sale. Of note, sample B8 is of decisive importance for the analysis and quality control of the vaccine.

The indicated time points at which samples are taken for analysis can be used as a "window" into the process. Clearly, these in-process samples differ from each other in their chemical composition. There are huge differences in the concentration of DNA contained, which can be up to three orders of magnitude.

In the following, the samples are briefly characterized with regard to their composition, and potential problems of analysis are mentioned (the cited DNA content is the result from ten consecutive analyses):
- B1: clearified virus harvest contaminated with proteins. DNA content between 100-4500 ng/ml, including high amounts of genomic DNA (up to 40 000 bp). An interfering influence of proteins is probable. The variable, high amount of DNA might lead to overloading of gels. The size range of the selected method must be suitable for molecules of interest.
- B2: 40% peak from the CS column, first concentration step. By cation exchange, DNA is partly eliminated. DNA content between 50-750 ng/ml, strongly contaminated with proteins and salt (elution from the column). An interfering influence of proteins is probable.
- B3: Second concentration step by diafiltration with an exclusion size of 500 kDa and buffer exchange. Protein concentrations of 1000-3000 µg/ml. Additionally, the sample comprises Tween 80, a non-ionic detergent which might influence results, in up to 5 µg/ml, which is not eliminated in the process up to the monobulk. DNA content is 500-5500 ng/ml, the highest concentration in the process. Difficulties as for B1.
- B4: The only difference to B3 is the addition of BPL and incubation for several hours. The content of DNA falls to 50-1200 ng/ml, and its characteristics are changed. Protein and detergent content like B3.
- B5: The membrane proteins have been solubilized by CTAB, and an ultracentrifugation has been carried out. CTAB content is between 800-3000 µg/ml. Detergent and protein concentration is similar to B3. The content of DNA falls to 5-26 ng/ml.
- B6: CTAB has been removed and the sample has been sterile filtrated. Detergent and protein content are reduced. The content of DNA is <1-15 ng/ml, which is not changed until the end of the process.
- B7: The final purification step with anion exchange chromatography has been carried out. Further substances like B6.
- B8: Monobulk. The material has been dia/ultrafiltrated and the buffer exchanged, resulting in a rise in the protein concentration by the factor approximately two. This is the most important sample with regard to analysis of DNA size. The protein content is 1500 µg/ml, the DNA content between less than 1 ng/ml (under conventional detection limits) to 15 ng/ml.

### 2. Sample Pretreatment

The sample pre-treatment can be varied, e.g., the order of steps can be changed, to achieve optimum results, depending on the sample analysed.

### 2.1 Proteinase K

For some experiments (see below), samples were digested with proteinase K. Interfering proteins, in particular nucleases are eliminated by this step.

20 µl of undiluted stock solution of the enzyme (2 mg/ml Proteinase K, Cat. No. 19133, Qiagen) were used for 1 ml of sample and incubated at 56°C (water bath, +/- 3°C) for 16 to 20 h.

It was found that better results were obtained if the digestion was carried out in the presence of SDS. 2% SDS-solution was added 1:1 to proteinase K (2 mg/ml). 50 µl of this mixture were used to digest 500 µl of sample. After mixing, the mix was incubated overnight (16 to 20 h) at 56°C).

### 2.2 DNA extraction

DNA was extracted with a kit for the sodium iodide method from Wako Pure Chemical Industries according to the manufacturer's instructions.

DNA extraction was tested for its suitability for concentration the samples. In particular, it was tested whether a lower buffer volume than recommended by manufacturer (500 µl) could be used for dissolving the DNA pellet. However, no complete dissolution of the pellet could be achieved using 50 µl of buffer (concentration by a factor of 10) for some tested samples. Thus, DNA extraction was not used for the concentration of samples in the following experiments.

DNA extraction was employed for purification of the DNA, e.g., from proteins and salts contained in the samples.

### 2.3 Concentration

Samples purified by DNA extraction (500 µl) were completely dried with a vacuum centrifuge (Speedvak) for 60 min. The dissolution of the pellet in 10 µl water was not successful. Raising the volume of solvent to 50 µl (concentration factor 10) only led to a partial success. The pellet from the samples with low DNA content (B5-B8) dissolved well, whereas the pellet from the other samples was not completely dissolved. The approach was not further employed for the following experiments.

Next, for concentration over a membrane, hydrophilic cellulose membranes with an exclusion size of 3 kDa and 10 kDa were tested (Microcon YM-3 and YM-10, Millipore). Maximal loading of 500 µl was used. Centrifugation was stopped when no fluid was left over the filter, however, the filter unit was maximally centrifuged for twice the recommend time. If there was still fluid above the filter, blocking of the filter was suspected and the matrix was not considered suitable for concentration of the respective sample. The dead volume of the used membrane filters was 10 µl. Thus, the theoretical concentration factor was 50.

The following parameters were used for centrifugation:
- 3 kDa membrane: 14000 g, 100 min, 25°C
- 10 kDa membrane: 14000 g, 30 min, 25°C.

For harvesting the concentrated sample, the filter unit was reversed and centrifuged in a fresh cap (1000g, 3 min, 25°C).

It was found that samples comprising proteins that were not pretreated were less suited for concentration by this approach. Both membranes were easily blocked in this case. The samples that had only been digested with proteinase K had to be centrifuged for the double time. With samples B1 and B2, still, the 3 kDa membrane was blocked. Much better results in a shorter time were obtained with the 10 kDa membrane. A comparative experiment (data not shown) demonstrated that, in the range of interest of between 100 and 1000 bp, no DNA fragments were lost, and that, roughly, a concentration by a factor of 40 was reached by membrane centrifugation.

### 3. Quantification of DNA

Concentration of nucleic acid can be determined based on the absorption at 260 nm (compared to absorption at 280 nm for proteins). However, detection limits are about 0,25 µg/ml, and several other substances also absorb at the same wavelength.

PicoGreen dsDNA quantification reagent (Molecular Probes) is based on a highly sensitive fluorescenct dye, with a detection limit of about 312 pg/ml dsDNA. The method is also suitable for detection of RNA or ssDNA, and the test is quickly done and rather cheap. However, the results vary by up to 30% depending on the concentration of contaminants in the sample. The results of this tests can be used to estimate the DNA concentration in a sample.

The determination of the concentration of DNA was preferably carried out with the Threshold^{®} System [Threshold^{®} Total DNA Assay Kit from Molecular Devices].

Calf thymus DNA was used as the standard. All samples from the process steps of vaccine production underwent the following pretreatment: Proteinase K-SDS-digestion at 56°C for 16-20 h (see above) and a DNA extraction with a commercial kit. Controls, standard and samples were denatured for 15-30 min at 105°C, so that the sample was present as ssDNA. All samples were incubated at 37°C for 60 min with the labelling reagent (Biotin-SSB (single stranded binding protein) and an antibody to DNA conjugated to urease). The obtained reaction complex was filtrated over a special filter unit, a nitrocellulose membrane, with bound streptavidin. After a washing step, the filter membrane is given into a potentiometric detector filled with substrate solution, and detection is started. The degradation of urea catalysed by the urease leads to a change in the surface potential. The curve of voltage over time is proportional to the concentration of DNA in the sample. The data are kinetically recorded and quantified on the basis of a standard curve.

### 4. Agarose gel electrophoresis in slab gels

Ready-to-use gel cassettes with ethidiumbromide (EtBr) were used according to the manufacturer's protocol (Invitrogen). Depending on the size range of the DNA, concentrations of 0.8%, 1.2%, 2% and 4% were employed. Separation was carried out at 60 V for 38 min. 1 kb+ DNA ladder standard (Invitrogen) in 1:200 dilution was used as a size marker.

To improve sensitivity, the gel cassette was opened and the gels stained with SYBR^{®}-Gold (Molecular Probes, Eugene, USA) for 30-45 min. The concentrate of the dye was diluted 1:10 000 in TE buffer according to the manufacturer's instructions (Molecular Probes, product information, revised 2001). The diluted solution was exchanged after 5 days. The gel was stained for 30-45 min. With SYBR^{®}-Gold, sensitivity was enhanced by a factor of 5 versus EtBr stain.

The production series with the highest concentration of DNA was used. The two samples with the highest concentration (B3, B4) were first analysed. As a reference for the concentration of DNA, the Threshold^{®} total DNA assay was used. The two samples were used either without pretreatment, or after digestion with proteinase K with and without DNA extraction. Significant differences with and without pretreatment were observed due to interactions between the proteins and the DNA (data not shown). Probably, larger complexes were formed which could not migrate into the gel. These were dissolved by the enzymatic digestion. The DNA extraction did not lead to a further advantage in this context. Thus, the improvement achieved by pretreatment could be confirmed for these samples.

The assay was repeated with all in-process samples from the same series. In this experiment, it was observed that DNA-extraction was helpful for obtaining clear results without interfering compounds (data not shown). The proteinase K treatment alone did not eliminate the high salt concentrations in the B2 sample and the interfering presence of CTAB (Cetyltrimethylammoniumbromid) in the B5 sample. DNA extraction eliminated all interfering substances.

However, the analyses showed that the concentration of DNA in the samples taken from later in-process steps or in the final product was not sufficient for detection with this method. To obtain clear results with regard to sensitivity, a dilution series of genomic DNA from MDCK cells was analysed (data not shown).

A minimal DNA concentration of 10 ng/ml was found. This demonstrates the necessity of establishing a more sensitive method of DNA analysis, as in the course of the production of the vaccine, such DNA concentrations can already be reached starting from sample B5.

The focus of analysis was on the size distribution of DNA. It was shown that in 1.2% agarose gels, fragments smaller than 100 bp were not detected in any of the samples. In contrast, in the capillary electrophoresis (see below) many fragments smaller than 100 bp were detected, in particular after treatment with Betapropiolacton.

To see this size range in the slab gels, 4% agarose gels were employed as opposite to 0.8% agarose gels. Two Mono Bulk (B8) samples were analysed. The 0.8% gel was not suitable for separation of the small fragments, which were retarded at the running front and appeared in a wrong size range. 4% gels were more suitable for the detection of the small fragments. Thus, agarose gels of two concentrations were required for analysis of the complete size range.

### 5. Polyacrylamide gel electrophoresis

Polyacrylamide allows production of clear and very thin gels (1 mm), which leads to very clear and sharp bands.

For polyacrylamide gel electrophoresis, gradient gels from 4 to 20%, TBE (Tris-Borat-EDTA buffer, Novex), were used. Separation was at 200V for 35 min. The gel was stained with SYBR^{®}-Gold. As a size marker, 1kb+ standard was used in a dilution of 1:200. A complete series of in-process samples (B1-B8) was analysed (data not shown).

In comparison to agarose slab gels, a better definition of the bands was achieved - in particular in the size range of interest below 100 kb. Analysis of fragment sizes over the complete range was better in agarose gel electrophoresis.

However, polyacrylamide gel electrophoresis in slab gels also does not achieve a sensitivity that allows determination of nucleic acids in the later in-process samples. Furthermore, the method is expensive and requires handling of toxic acrylamide.

### 6. Capillary gel electrophoresis

### 6.1 Basic setup of the separation system

P/ACE MDQ Molecular Characterisation System (Beckman Coulter) was used for separations. An argon ion laser with a wavelength of excitation of 488 nm and an emission filter of 520 nm was used for detection. The capillary used in this system was a fused silica capillary with an inner diameter of 100 µm neutrally coated on the inner side (eCap dsDNA kit, Beckman Coulter). Other materials can be used for the capillary. The neutral coating (polyacrylamide-based hydrophilic surface) nearly completely suppresses the electroosmotic flow (EOF) and minimizes interactions of the analyte with the inner surface of the capillary. Both effects improve definition of bands and reproducibility.

A double coating was used for covering the inner surface of the capillary. The first coating is bound on the free silanol groups of the fused silica capillary and covered these. The second coating of hydrophylic polyacrylamide reduced hydrophobic interactions. This coating is stable for about 200 separations or more, wherein a detoriating power of separation can be used as an indicator for loss of stability.

To improve mechanical stability of the capillary, the outer surface of the capillaries is covered with a polyimid coating of about 10 µm, which is removed for detection, as the coating is not transparent to UV light.

The complete length of the capillaries that were initially used was 60 cm with 50 cm to the detector. In the course of optimization, the length was changed to 50 cm with 40 cm to the detector. All separations were carried out with inversed polarity, i.e., with the detector at the anode end. As separation buffer, a Tris-Borat buffer with a pH of 8.9 was used. The buffer belongs to the group of non-crosslinked physical gels with low viscosity which are most suitable for the method of the invention. It has a dynamic pore structure and is not sensitive to heat. Before use, the buffer was filtered with a 0.45 µm syringe top filter and degassed for 10 min in an ultrasound bath to prevent formation of bubbles.

The gel matrix was exchanged after each use. Before the first separation, a new capillary was conditioned for 10 min at 136 kPa (20 psi (pounds per square inch), 1 psi corresponds to 6894,75728034313 Pa) with fresh buffer. Before each run, the capillary was filled with fresh buffer for 5 min at 136 kPa. After each separation, the capillary was washed with the buffer for 5 min at 204 kPa. Preferably, no washing step with distilled water was carried out, as, this way, conditions in the capillary were maintained more constant, leading to a longer life of the inner coating of the capillary. With a volume of the capillary of 3.9 µl, the required amount of buffer was negligible. The separation temperature of 20°C suggested by the manufacturer was used.

The parameters of the LIF-detector (laser induced fluorescence) were set as follows:
- dynamic range: 100 RFU
- filter: normal
- peak width 16-25
- data rate 4 Hz.

The algorithm for integration was set at "standard CE" and carried out with reference to migration time. The calibration of the LIF-unit was carried out according to the manufacturer's protocol [P/ACE MDQ LIF Detector Manual, 718113-AB, Beckman Coulter, Fullerton, USA] and the obtained factor of 1.1 was used.

### 6.2 Optimization

From one series of in-process samples, the two samples with the highest DNA content were used (B3 and B4). The samples were purified by DNA-extraction as described above.

At first, the standard method according to protocol [Care and Use instructions for eCAP dsDNS 1000, 726412-C, Beckman coulter] was used. A field intensity of 200 V/cm, hydrodynamic injection over 10 s at 3 kPa and a total duration of 20 min were used. Parameters for loading the column were studied with regard to their suitability for detecting genomic DNA with high sensitivity, and optimized for the two selected samples.

Hydrodynamic injection is preferred over electrokinetic injection. Hydrodynamic injection renders it possible to take several samples from one sample vial and thus reduces the required amount of sample for several analyses. Thus, from a sample of 50 µl volume, 10 analyses could easily be done, whereas the alternative electrokinetic injection requires tenfold higher sample volumes for the same number of tests to achieve similar reproducibility. Hydrodynamic injection at 103 kPa and 30 s led to the best results (data not shown).

The effect of betapropriolacton treatment of the vaccine preparations could clearly be observed. In contrast to the B3-sample, no peak was detected for genomic DNA, but a degradation of DNA to small fragments was observed (data not shown).

In B3-samples stored for a longer period of time, a degradation of genomic DNA to smaller fragments was also observed. For example, a B3 sample from a current fermentation was compared with a B3 sample (from a fermentation with the same virus strain) stored at 4-8°C for 4 months. Degradation, which is probably due to enzymatic activity of nucleases in the sample, was strongest for DNA fragments of middle length (20-25 min). Due to this effect, an immediate treatment of samples taken for analysis with Proteinase K is necessary, if nucleases are present in the sample and degradation is to be prevented. Proteinase K, an endoproteinase with low specificity, degrades and inactivates all proteins.

This effect was not observed with later in-process samples (B4-B8). It can be safely assumed that the enzymes do not survive the downstream process.

The combination of parameters with the best results was maintained for further analysis.

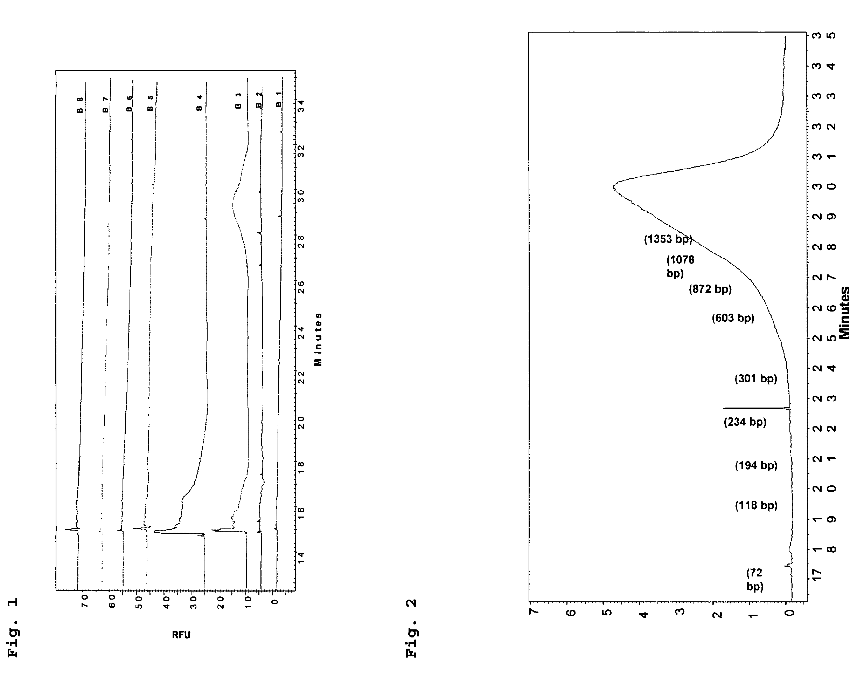

A complete series of in-process samples was purified by DNA-extraction and analysed to see whether nucleic acids in all samples could be determined (Fig. 1).

In these preliminary experiments, several problems arose:
mainly, the sensitivity of the analyses was not sufficient to analyse nucleic acids in all samples. Furthermore, as anticipated, the DNA did not appear in sharply separated peaks, which made an exact determination of size difficult. Additionally, in the determination of complete DNA with regard to the first three process samples, there was no correlation with the Threshold^{®} Assay as a reference test, which indicated DNA concentrations about five times higher for B3 than for B1. However, as can be seen from Fig. 1, the electropherogram of the B1 sample with 980 ng/ml DNA appeared more similar to the B7 sample with 12 ng/ml.

### 6.3 Calibration

For qualitative analysis, a suitable size standard was necessary. With the target of achieving a complete separation, a 10 bp marker (DNA ladder Cat. No. 10821-051, Invitrogen), a 1 kb marker (eCAP 1000 dsDNA test mix, φX-174 HaeIII, Cat. No. 477414, Beckman Coulter) and a 2 kb marker (20.000 dsDNA test mix λDNA/HindIII fragments, Cat. No. 477483 Beckman Coulter) were compared. Criteria for assessment were separation of the basis lines of two neighbouring DNA fractions and the form of the peaks with regard to their suitability for integration. Duration of the separation process was only of minor interest. Focus was on the appropriate separation of the complete range of DNA lengths present in the sample. The calibrations with the markers showed that with the selected matrix for separation, the area of linearity is left at about 1000 bp of length. In the lower range it extends to about 10 bp (data not shown).

As expected, the power of differentiation decreased for DNA molecules over 1000 bp, making the separation matrix less suitable for large molecules. MDCK genomic DNA was analysed by capillary electrophoresis (Fig. 2). The broad form of the MDCK peak shows that the DNA was not separated, and could not easily be analysed by size. However, the important point is that the presence of genomic DNA could be detected, which was sufficient for the problem underlying the invention. Under the selected conditions, 35 min were necessary for good detection of genomic DNA.

The most exact results with regard to quantity are obtained if a size standard and the sample are co-injected. It was tested whether it is possible to separate all three size standards mentioned above in a single run (data not shown). A good separation of peaks was obtained. If the length of DNA in a sample that is to be analysed is known, co-injection seems to be a good method for analysis. However, this option is not preferred in case the size is not known or if the size corresponds to the size of a marker fragment, as the risk that a sample peak and a marker peak are superimposed is high.

Thus, it is preferred that at the beginning and the end of each series of separations, a calibration with a standard is carried out. For the analysis underlying the invention, a 1kb standard (smallest band 72 bp) was preferred, and used for all further experiments. As the separation in the selected matrix is linear to the range of 10 bp, extrapolation can be used for determination of sizes under 72 bp.

It is preferred to store the standard in concentrated form as a stock solution (e.g., 200 µg/ml). Dilutions should be freshly prepared each day.

### 6.4 Length of separation and field intensity

For the experiments described above, the separation was carried out after hydrodynamic injection at 7 kPa for 15 s, 200 V/cm at reversed polarity, a capillary length of 50 cm to the detector and separation temperature 20°C. With these settings, the 1 kb standard could be separated in about 35 min.

It was however found that much better results, in particular a better efficiency of separation and a shorter duration of analysis were obtained with 250 V/cm. Higher values, such as 300 V/cm, significantly decreased the resolution. Thus, separation at 200 to 275 V/cm, in particular at 225 to 265 or 240 to 260 V/cm is preferred. The following experiments were carried out at 250 V/cm. With regard to the duration of analysis, the length of the capillary was selected to be 40 cm to the detector. This change did not affect efficiency of separation.

By these measures, the duration of analysis could be shortened from 35 min to 23 min.

To further reduce the required time, the effect of temperature on the duration of the separation was analysed. It could be shown that a separation temperature of 30°C reduced the separation time, however, it decreased the separation of the peaks of 271 bp and 281 bp. Thus, a separation temperature of 16 to 25° C is preferred.

In case a higher separation temperature is chosen, further adaptation of the parameters or the matrix should be carried out.

### 6.5 Improving the quality of the analysis

Several times, contaminants were carried over from samples. Additionally, there were significant variations in the results from repeated analysis of the same sample.

To address this problem, after injection of the sample, a brief hydrodynamic injection with water was introduced. Surprisingly, this step alone already significantly improved reproducibility of the analysis.

The results were further improved by washing the ends of the capillary before and/or after each contact of the capillary with a sample vial. Washing was preferably carried out by dipping the capillary/electrode ends into a vial with water.

To minimize contaminations, different vials of water were used for washing and for the hydrodynamic injection of water.

The water was preferably purified water, in particular destilled or deionised water or bidestilled water.

Furthermore, results were improved if the coating on the outer side of the capillary was removed on a length of about 2 mm at the ends of the capillary, e.g., by flaming.

### 6.6 Sensitivity

Sensitivity problems were evident from the results of the agarose gel electrophoresis and the initial results of capillary gel electrophoresis, in particular when the Mono Bulk samples with DNA concentrations in the range of pg/ml were analysed.

A wide selection of dyes for DNA is available, e.g., intercalating dyes. Dyes detectable by fluorescence, in particular under argon laser, are most suitable for use in the methods of the invention. Ethidiumbromid or derivatives thereof are preferred, in particular EnhanCE dye from Beckman Coulter. Preferably, a surplus of the dye is added to the separation buffer and stains the DNA on column. Thus, there is no need to contact the DNA with the dye before injection. Comparative experiments showed that this method is superior to staining before injection, as it leads to a very "quiet" base line and high selectivity.

Use of the dye led to a signal enhanced by the factor of ten (data not shown). The intercalation made the DNA molecules longer, which slightly increased the duration of the separation. The form of the peaks is enhanced, which seems to be due to altered electrical charges of the molecules. A slightly higher, constant background signal was observed. Based on these experiments, it is preferred to add an intercalating dye based on an ethidiumbromide derivative suitable for LIF detection to the separation buffer, in particular EnhanCE, namely, EnhanCE in a concentration of 1-5 µl, preferably 3 µl dye per ml separation buffer.

The buffer was prepared by filtration through a sterile membrane (0,22 µm), degassing in an ultrasound bath or a vacuum concentrator for 10 min, followed by addition of the dye. After addition of the dye, the buffer should neither be filtrated nor degassed due to the dye's sensitivity to these treatments. Thus, the dye should be mixed well without introduction of air. The most suitable method is carefully and repeatedly taking up the solution with a pipette. The preparation is also sensitive to light. In dilution, the dye is degraded after about 10 hours, which limits the total duration of a separation sequence. It is technically possible to separate about 40 samples in one series. However, with a total duration of 40 min per run, the dye is not stable enough. Thus, it is preferred to test up to 15 samples in one series.

### 6.7 Sample concentration

For samples with a very low nucleic acid concentration, such as some mono bulk samples, the sensitivity of the method was still not sufficient for determination of nucleic acids.

By concentration of the samples via membrane centrifugation, in particular after DNA extraction, the concentration of DNA could be significantly enhanced. After DNA extraction, the volume of the sample was 500 µl. The minimal volume for CE is about 10 µl. Thus, the maximal concentration factor was 50, which could be used by membrane centrifugation, as described above in detail. However, due to the variable volume that was obtained with this method, the concentration factor could not be exactly determined.

A second possibility to improve sensitivity is an online concentration before running the sample on the capillary [Osbourn et al., On-line preconcentration methods for capillary electrophoresis, Electrophoresis 21 (2000), 2768-2779; Quirino et al., Sample stacking of cationic and anionic analytes in capillary electrophoresis, Journal of Chromatography A, Vol. 902 (2000), 119-135]. There are two possibilities, focussing the sample with "field amplified sample stacking" (FASS), a hydrodynamic method, or focussing the samples with electrokinetic injection, "field amplified sample injection" (FASI).

For FASS, the sample has to be dissolved in a sample buffer of lower conductivity than the running buffer itself or, in the simplest case, in water. The sample is then injected hydrodynamically. At the interface between the sample solution and the buffer, the molecules are accelerated in direction to the interface under voltage, and thus, the sample is focussed. This effect can be enhanced by pre-injection of a short plug of a highly concentrated buffer before injection of the sample.

FASI employs an electrokinetic injection from a first vial with sample solution with low conductivity into the capillary, which is filled with buffer. In theory, a high difference in concentration leads to a strong focussing.

An alternative FASS-injection with a high volume was described in [Osbourn et al., On-line preconcentration methods for capillary electrophoresis, Electrophoresis 21 (2000), 2768-2779], which is based on EOF. Experimentally it was shown that the method is not suitable for use with the coated capillary described above, as the EOF was not sufficient (data not shown). It was tried whether the lack of EOF could be compensated for by pressure on the outlet side. With this method, the sample plug could be moved backwards under voltage. However, there was no orientation of changing currents to find the optimal time for stopping the focussing, so it was hard to get reproducible results.

Further experiments concentrated on sample focussing without use of EOF.

The method so far described was adapted to electrokinetic injection of the sample with pre-injection of water. It was found that the sensitivity of the method could be significantly enhanced by this step.

To maximize concentration of the sample, the online method of sample focussing with electrokinetic injection was combined with the concentration of samples by membrane filtration.

The following protocol describes sample treatment and analysis under the conditions leading to the best results:
In-process samples from the preparation of influenza vaccine were digested with proteinase K (e.g., 1 h at 56°C or over night), followed by DNA-extraction. The resulting sample (e.g., 500 µl), was concentrated by membrane centrifugation (e.g., over a centricon membrane with a cut-off weight of 10 kDa).

A volume of 10-25 µl was separated by capillary gel electrophoresis under the following conditions:
- Hydrodynamic pre-injection of water, e.g., at 3 kPa for 5 s,
- Electrokinetic sample injection, e.g., at 10 kV for 30 s,
- Hydrodynamtic post-injection with water, e.g., at 1 kPa for 5 s,
- Separation, e.g., at 250 V/cm for 35 min, with the separation buffer comprising a dye for staining the DNA (e.g., EnhanCE at 3 µl/ml).

Size markers were run at the beginning and end of each series of separations under the same conditions.

As mentioned above, the treatment of samples with proteinase K and the DNA extraction was only required for samples comprising significant amount of protein, such as the B3 sample, and/or high salt concentrations. However, for better comparison of the samples, all samples were treated the same way.

According to this protocol, several fermentation steps of vaccine preparation were completely analysed. At the beginning of the fermentation process, the DNA was present as genomic DNA, at the end, it was strongly damaged by betapropriolacton treatment. Therefore, broad peaks, sometimes over several minutes occurred, which made it difficult to exactly allocate sizes.

### 6.8 Analysis of results

Time or mobility windows were employed for the analysis of results obtained as previously described.

It is preferred that mobility instead of time is used for identification of peaks. In this approach, small changes in results obtained with each run can be compensated. In particular, in time-dependent peak identification, the slow hydrolysis of the polyacrylamide network led to a slight shift backwards on the time axis from experiment to experiment. If the peaks left their defined time window, it was harder to identify them.

Mobility is a parameter that quantitatively defines how charged particles migrate in an electrical field. Components with high mobility move more quickly than components with low mobility. Mobility is not constant and depends on the parameters chosen for analysis. Change of the parameters of separation, such as changes in the voltage provided or the slow hydrolysis of the matrix, can be compensated by using a standard of defined mobility. One precondition is a stable pH, which is guaranteed by use of the buffer.

Analysis by mobility requires a reference point, for which the first standard peak (72 bp) was selected.

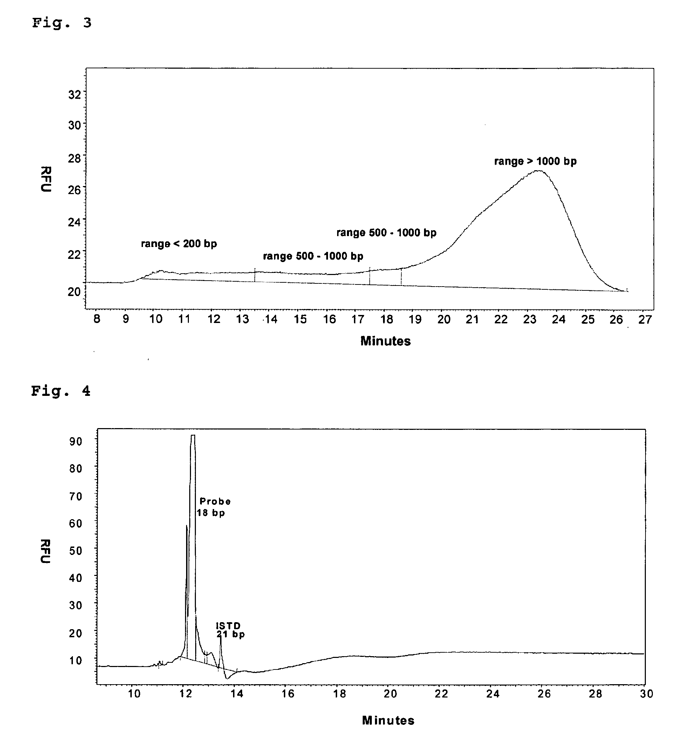

The software for analysis, 32 Karat^{®}, allows the analysis of time windows which correspond to selected size ranges. Peaks in this size range were summed up and their area was calculated. Thus, in relation to the complete area, the proportion of DNA in a certain size range could be determined (Fig. 3).

In an analysis of the B8 sample containing the lowest concentration of DNA (<1ng/ml as determined with the threshold^{®} assay), DNA fragments of 18 to 21 bp could be detected. The method of the invention could thus be used to show that no nucleic acids longer than 21 bp were contained in the sample.

### 6.9 Determining the limit of sensitivity

A dilution series of 1 kb standard was analysed to determine the sensitivity limit of the method of the invention. Only peaks with a signal to noise ratio of more than 3 were considered peaks. A minimal DNA concentration of the standard of 100 pg/ml was determined. These results cannot be directly compared to the minimal concentration of DNA detected by agarose gel electrophoresis, which was determined with a single DNA fraction. In contrast, the 1 kb standard contained 11 fragments, the quantitative composition of which was not calculated. To be able to estimate the sensitivity with regard to the smallest DNA fragment (72 bp) the percentage of the area (0.69%) of the corresponding peak of the complete area was calculated. The concentration of the 72 bp fragment was determined to be about 0.7 pg/ml.

In comparison to agarose gels, the sensitivity of the method based on capillary gel electrophoresis is higher by a factor of 14 000! Of note, this sensitivity only applies to the detection of a DNA fragment of a single length. This explains that for detection of the DNA in the sample comprising 1 ng/ml, concentration steps were required.

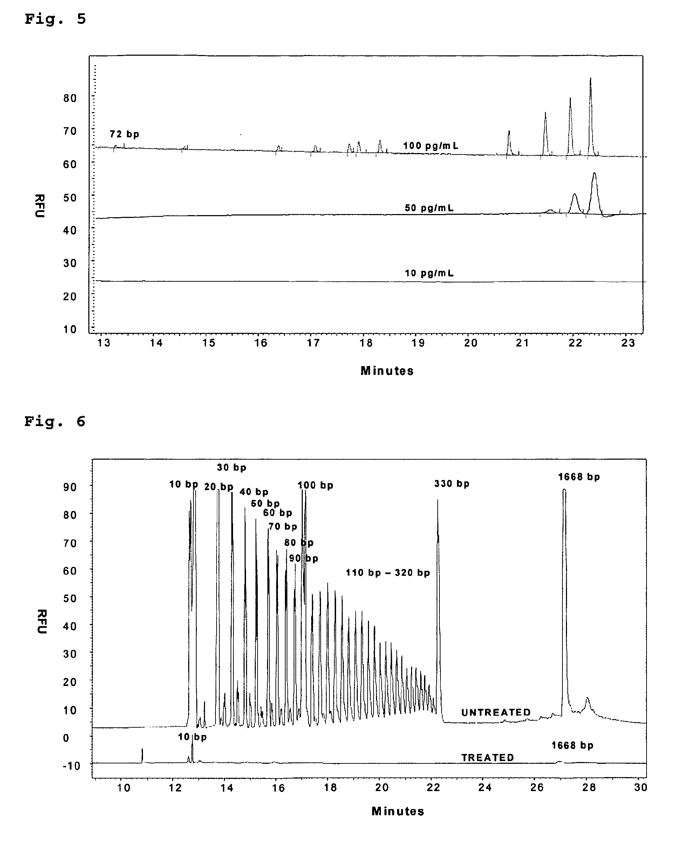

Sensitivity depends on the length of the DNA fragments. In longer fragments, more dye can intercalate, which leads to a stronger signal (Fig. 5).

### 6.10 Minimizing the cost of the analysis

Several issues, which do not directly affect separation, but greatly influence the experiments and their costs were investigated in the course of the studies.

The most expensive reagent used is the separation buffer. As suggested by the standard protocol of the manufacturer (Beckham Coulter), 2 ml storage vials were used. While the capillary's temperature is regulated, the storage vial with the separation buffer, into which the electrode and the end of the capillary are immersed, is normally at room temperature. This leads to fast hydrolysis of the buffer, so that it loses its sieve effect. Additionally, the dye is not stable over more than one day - and the danger of carrying over contaminations from samples also made it preferable to exchange buffer vials every day.

It was found that 200 µl PCR vials could advantageously be used as storage vials for the separation buffer in capillary gel electrophoresis. On the basis of the detected currents, the optimal capacity of the buffer was determined to be maximally 5 separations per pair of storage vials of separation buffer. No decrease in the power of differentiation was observed. An exemplary calculation of costs demonstrated that significant savings can be achieved. Thus, a test kit at the price of 900€ comprises 60 ml of separation buffer. With the method as proposed by the manufacturer, about 150 runs can be carried out. The use of smaller storage vials renders 600 runs with the same amount of separation buffer possible. Thus, costs are lowered to a forth by this small but effective measure.

A kit for capillary gel electrophoresis comprising separation buffer in vials of 200 µl is therefore provided. Preferably, the kit includes a suitable dye, e.g., EnhanCE dye and standard, e.g., 1kb Standard from Beckman Coulter (consisting of Hae III restriction digest φ174 DNA containing 11 fragments from 72 bp to 1,353 bp), and optionally, coated capillaries as described above.

### 6.11 Troubleshooting

In the course of the experiments, sporadic power failures were observed. If that was the case, the run could not be used for analysis. The problem was traced to microbubbles forming in the capillary during separation. If the degassing step in the preparation of the separation buffer was dropped, the effect appeared more frequently. Degassing for a longer time decreased the frequency of the problem. By applying pressure of about 34 kPa to both ends of the capillary during the separation phase, the problem was effectively circumvented.

One danger in working with viscous gels is evaporation of the solvent, which can lead to hard crusts at the lids of the storage vials of the separation buffer, on the outer side of the capillary and the electrode. The lids of the storage vials have openings for the capillary. If crusts form at this place when the capillary is withdrawn, this can lead to breaking of the capillary in the next experiment. The deposition on the electrodes can lead to undesirable creeping currents and negatively affect the separation of peaks. Consequently, a daily exchange of lids and cleaning of the electrode and the outer side of the capillary is recommended.

In the course of the experiments, it was observed that the samples are diluted by injection of samples from the vial (data not shown). Thus, several sample aliquots were provided if multiple determinations of the same sample were planned.

### 6.12 Influence of betapropriolacton treatment

It was investigated if betapropriolacton (BPL) had any effects on the separation of DNA by the method of the invention.

In the fermentation process of the vaccine, BPL inactivates DNA by alkylation, wherein the BPL molecule reacts with the nucleophilic centers of the DNA, leading to crosslinking and denaturation. Upon longer contact, single strand breaks appear and single bases may be lost. If the contact is sufficiently long, the DNA undergoes fragmentation and loses its biological activity.

The three size markers employed in the experiments (10 bp, 1 kb and 2 kb standards, see above) were treated with BPL for 16 h, like the vaccine preparation. A general lower intensity of DNA was observed (Fig. 6). Peaks in small concentrations completely vanished. This experiment showed that BPL does not affect the characteristics of separation of DNA. As long as DNA was present, the dye could intercalate and lead to a signal. Shorter fragments seem to be more rapidly decomposed than longer fragments.

### 6.13 Better precision with an internal standard

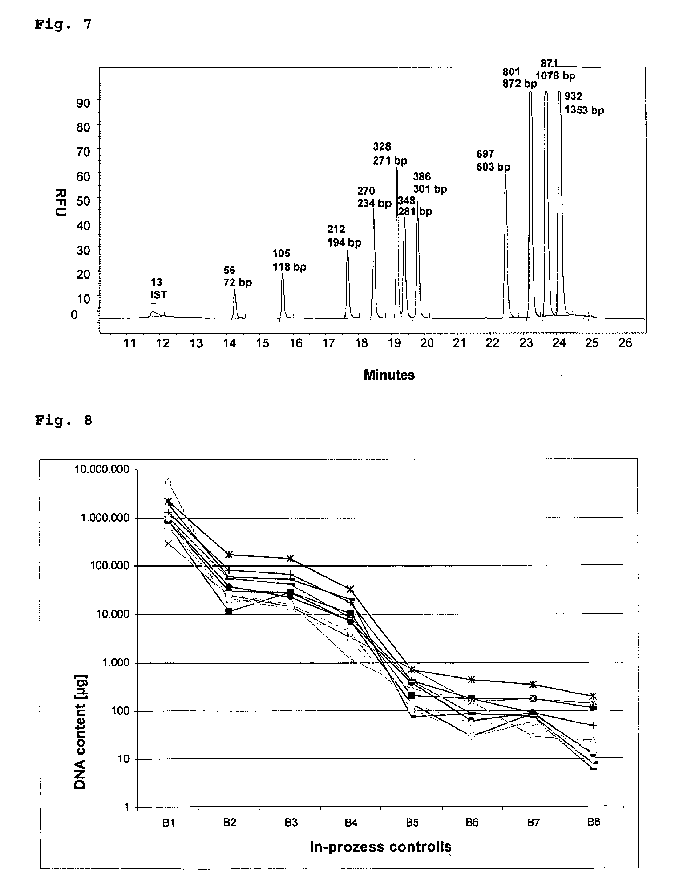

Precision of the analysis can be improved with internal standards (ISTD). A suitable substance is loaded before each sample injection and is employed as a reference for identification of peaks, so that external influences are eliminated. The standard should belong to the same class of substances as the sample. To minimize interference with the sample, the ISTD should appear before the sample peaks. A 23 base primer (ssDNA) was selected, which was strongly detected. This peak was allocated the mobility of "-10.000", however, the exact value depends on the parameters of separation, and e.g., may differ with use of different software. The negative algebraic sign is explained by the reversed polarity (cathode at the inlet). Accordingly, in an exemplary experiment, the peaks of a size standard were allocated a relative mobility in comparison to the ISTD. With this method, calibration is less sensitive to outer influences. The concentration of the ISTD was 10 µg/ml, and it was injected hydrodynamically with 21 kPa for 5 s (Fig. 7).

### 6.14 Summary

The limit of detection achieved with the method of the invention was about 9 pg/ml dsDNA for a fragment of 72 bp. This corresponds to 100 zmol (10⁻²¹ mol). This corresponds to 90210 molecules in a milliliter for dsDNA with a molecular weight of 660 g/ml. Only 100 µl were used for analysis. With electrokinetic injection over 30 s it is assumed that all molecules present in the vial have migrated into the capillary. Thus, about 9000 dsDNA molecules of this size are sufficient for detection.

In detail, the preferred method of the invention comprises the following steps:
1. The sample is digested with proteinase K for 1 h at 56°C, preferably directly after it has been taken. Alternatively, the sample can be stored at or below -20°C until all relevant samples can be treated together. It is preferred to treat the sample with proteinase K in the presence of SDS, as described above.
2. The DNA from the sample is extracted (e.g., with a DNA extraction kit commercially available from Wako), and dissolved in purified water or buffer. In particular for concentrated samples, the volume should be identical to the volume before DNA extraction.
3. Samples can be concentrated over a membrane with an exclusion size of 3 kDa or preferably 10 kDa, e.g., a hydrophilic cellulose membrane, such as the Centricon filter from Millipore. No fluid should be visible over the filter after centrifugation. Suitable Parameters for concentration are, e.g., 14.000 g, 15 min, 20°C, and for harvesting the concentrate, 3.000 g, 3 min, 20°C.
   It was shown by comparative experiments that step 1 is not required if the amount of protein in the sample is negligible, e.g., in samples B6-B8. Here, no difference was observed between samples with and without digestion and with and without DNA extraction (data not shown). Step 2 is not required if the amount of salts and other contaminants does not interfere with analysis, and step 3 is not required if the concentration of nucleic acids in the sample is sufficient for detection without previous concentration. However, it is preferred that different samples that are to be compared are treated the same way.
   If only, e.g., monobulk samples or comparable samples are to be analysed it is recommended to dispense with the proteinase K digestion and DNA extraction, so costs are saved and the time of analysis per sample is reduced from about 3 hours to about 30 min.
4. The capillary electrophoresis system, e.g., P/ACE MDQ Molecular characterization system, Beckman Coulter, is prepared as follows:
   - detection with laser induced fluorescence at 488 nm, emission 520 nm.
   - Capillary: neutrally coated
   - Capillary size: 50,2 cm, total length 40 cm to the detector, inner diameter 100 µm
   - New capillaries are conditioned with separation buffer, e.g., for 10 min. Of course, a comparable system and capillary can be used.
5. With regard to the control software, the following settings should be entered.
   - integration modus "Standard CE"
   - peak identification based on mobility
   - separation temperature 20°C, storage temperature for samples 4°C
   - starting run after the temperature of separation has been reached
   - control of run with reference to current
   - dynamic range 100 RFU
   - filter: normal
   - peak width 16-25
   - data rate 4 Hz.
6. The separation buffer is prepared (Tris-Borat-Gel buffer, pH 8,9 (e.g., from Beckman Coulter, which is available lyophilized and is dissolved for 24 h at 4-8°C under continuous mixing and is stable for 30 h at 4-8°C). The required amount of buffer is purified over a 0.45 µm filter membrane and degassed for about 10 min in an ultrasound bath. 2 ml separation buffer is kept without dye as a washing buffer. To the rest, 3 µl of dye are added for 1 ml separation buffer and the mix is homogenized by careful pipetting, ensuring that no air is introduced. The separation buffer is portioned in 200 µl PCR vials. The preparation is sensitive to light and can be stored for 10 h.
7. Dilutions of standards are prepared. In particular, if the sample is in water, purified water is used for this purpose. The concentration of the standard is 100 ng/ml. "eCAP 1000 dsDNA Test Mix φ174 HaeIII" is recommended as size marker. The standard is to be tested under the same conditions as the sample, preferably, at the beginning and the end of each series of runs, on the same day.
8. The following sequence of events can be determined for a sample or calibration run:
   - Equilibrating the capillary with separation buffer comprising dye, 5 min at 136 kPa
   - Washing by dipping the ends of the capillary and electrodes into water, 0 kPa, 1 s
   - Hydrodynamic injection of water, 21 kPa, 5 s
   - Hydrodynamic injection of ISTD, 21 kPa, 5 s
   - Washing by dipping the ends of the capillary and electrodes into water, 0 kPa, 1 s
   - Electrokinetic injection of sample, 10 kV, 30 s at reversed polarity (cathode at the inlet)
   - Washing by dipping the ends of the capillary and electrodes into water, 0 kPa, 1 s
   - Separation with 12 kV and 30 min with reversed polarity (cathode at the inlet), preferably, after every fifth sample, the vials of separation buffer are exchanged
   - Collection of data for about 30 min
   - Washing step of the capillary with separation buffer without DNA dye, 204 kPa, 5 min
   - Washing by dipping the ends of the capillary and electrodes into water, 0 kPa, 1 s
   - Capillary is brought into the starting position in water
   - End.
9. The data is collected and analysed with 32-Karat Software. Each analysed peak should be recognized by the software. However, if necessary, manual integration may be done.
   Preferably, the in-process samples from vaccine production are analysed for the presence of DNA of four size ranges. Thus, four groups are added, which, after calibration, are allocated to certain time/mobility windows. These can correspond to the following size ranges: <200 bp, 200-500 bp, 500-1000 bp and >1000 bp. The percentage of the area of the peaks is calculated.
10. If required, a report is drawn up.
   The reproducibility of data achieved with this method was tested with two methods. The relative percentage of standard deviation for peak identification was 1.28%. For integration of the area, this value was 4.04%. Thus, the method led to highly reproducible results.

### 6.15 Discussion of errors

Systemic changes in measurement are compensated by the use of an internal standard with each measurement, as all changes in conditions that affect the measurement also affect the ISTD.

The reproducibility of data achieved with this method was tested.

The area of confidence in which the internal standard peak must be recognized as such has to be calculated. The relative standard deviation of the migration time of this peak was 0.24 min for ten measurements. The value of 0.24 min was defined as the time window for the ISTD for the software. This value was thus introduced in the program settings, rounded to 0.5 min. For the software, this means that the peak has to appear 0.25 min before or after the defined time, otherwise, the measurement is not analysed. The relative percentage of standard deviation for peak identification was 1.28%, which was very low (this means a deviation of +/- 12 bp for a size of 1000 bp, which is negligible).

For integration of the area, the relative percentage of standard deviation was 4.04%.

Thus, the method led to highly reproducible results.

It is decisive to calibrate with a size standard for associating the sample peaks with the respective size range. The correlation coefficient is a measure of linearity of the calibration points, and it was determined to be 0.99 for the 10 bp standard. For the 1 kb standard it was 0.95. These values are sufficient.

### 6.16 Size distribution and concentration of DNA in the samples

The results for size distribution from the investigated fermentations are presented as Table 2 for five of the in-process samples. The size distribution is presented as Table 2.

**Table 1: Size distribution as the average of five fermentations. Samples were treated with proteinase K and DNA was extracted. DL= detection limit: 0.7 ng/ml**

| In-process sample | <200 bp | 200-500 bp | 500-1000 bp | >1000 bp |
|---|---|---|---|---|
| B1 | 25% | 30% | 16% | 34% |
| B3 | 25% | 12% | 6% | 55% |
| B4 | 79% | 29% | 5% | 4% |
| B6 | 69% | 60% | <DL | <DL |
| B8 | >99% | <DL | <DL | <DL |

The presentation of the size distribution does not take the DNA concentration present in the samples into account. As described above, the 99% in B8 relate to an average DNA concentration of 10 ng/ml, while in B1 samples, DNA concentrations may be as high as 4000 ng/ml.

Thus, the concentration of DNA was reduced by several orders of magnitude during the process of vaccine preparation. The reduction is more apparent when the absolute amounts of DNA are presented (Fig. 9).

Two process steps could be identified that most significantly contributed to this reduction. The first is the cation exchange chromatography, and the second, surprisingly, the treatment with CTAB. To this end, the treatment with CTAB is preferably followed by diafiltration.

Treatment with BPL, as previously discussed, contributed most to the reduction of DNA size. By testing amplification of, e.g., beta-actin by PCR, it was investigated if DNA in the samples could still be biologically active after BPL treatment (data not shown). No amplification was observed for any of these samples, indicating that no complete, functional template was present in the samples. Further experiments with probes, some of these directed to known oncogenes also indicated that no functional DNA survives the fermentation process (data not shown), and thus confirm the results obtained with the method of the invention.

**Table 2 Size distribution of DNA from five fermentation steps. Samples were treated with proteinase K and DNA was extracted. DL= detection limit: 9 ng/ml**

| **In-process control** | **<200 bp** | **20bp00 bp** | **500-1000 bp** | **>1000 bp** |
|---|---|---|---|---|
| **A/New Caledonia (lot OOCL110401)** | | | | |
| Clarified fermenter harvest (B1) | 12% | 71% | 10% | 7% |
| Column eluate after diafiltration (B3) | 8% | 9% | 11% | 72% |
| Concentrate after BPL inactivation (B4) | 47% | 47% | 6% | <DL |
| Concentrate after CTAB splitting (B6) | <DL | <DL | <DL | <DL |
| Monovalent bulk material (B8) | >99.9 | <DL | <DL | <DL |

| **A/Panama (lot OOCN110402)** | | | | |
|---|---|---|---|---|
| Clarified fermenter harvest (B1) | 38% | 6% | 3% | 53% |
| Column eluate after diafiltration (B3) | 65% | 21 % | 1 % | 13% |
| Concentrate after BPL inactivation (B4) | 78% | 22% | <DL | <DL |
| Concentrate after CTAB splitting (B6) | >99.9 | <DL | <DL | <DL |
| Monovalent bulk material (B8) | >99.9 | <DL | <DL | <DL |

| **B/Jiangsu (lot OOCC110403)** | | | | |
|---|---|---|---|---|
| Clarified fermenter harvest (B1) | 7% | 25% | 16% | 52% |
| Column eluate after diafiltration (B3) | 5% | 2% | 1 % | 92% |
| Concentrate after BPL inactivation (B4) | 78% | 18% | <DL | 4% |
| Concentrate after CTAB splitting (B6) | 17% | 83% | <DL | <DL |
| Monovalent bulk material (B8) | >99.9 | <DL | <DL | <DL |

| **B/Jiangsu (lot OOCC110405)** | | | | |
|---|---|---|---|---|
| Clarified fermenter harvest (B1) | 29% | 18% | 15% | 38% |
| Column eluate after diafiltration (B3) | 25% | 3% | 2% | 70% |
| Concentrate after BPL inactivation (B4) | 96% | <DL | 4% | <DL |
| Concentrate after CTAB splitting (B6) | >99.9 | <DL | <DL | <DL |
| Monovalent bulk material (B8) | >99.9 | <DL | <DL | <DL |

| **A/New Caledonia (lot OOCL110406)** | | | | |
|---|---|---|---|---|
| Clarified fermenter harvest (B1) | 38% | <DL | 37% | 18% |
| Column eluate after diafiltration (B3) | 20% | 26% | 16% | 27% |
| Concentrate after BPL inactivation (B4) | 97% | <DL | <DL | <DL |
| Concentrate after CTAB splitting (B6) | 61% | 36% | <DL | <DL |
| Monovalent bulk material (B8) | >99.9 | <DL | <DL | <DL |

### 6.17 Compliance of the vaccine with regulations

For a regulatory maximum of 100 pg DNA/dose of vaccine, 200 pg/ml is the highest allowable concentration of DNA in a 0,5 ml dose of vaccine. As the DNA can be qualitatively analysed with a sensitivity of at least about 9,0 pg/ml, the present invention provides a method of quality control of a vaccine for contaminations with DNA, which employs capillary gel electrophoresis.

### Figures

**Fig. 1** Analysis of in-process samples of a fermentation of influenza vaccine with heavy DNA contamination by capillary gel electrophoresis with hydrodynamic injection at 103 kPa for 30 s, no dilution of samples. The scale for samples B3 and B4 is smaller by a factor of five.

**Fig. 2** Analysis of MDCK genomic DNA in comparison to the 1 kb standard by capillary gel electrophoresis with hydrodynamic injection at 7 kPa for 10 s. The numbers in parenthesis show the normal time points of the calibration peak.

**Fig. 3** Analysis of a B3 sample with the method of the invention as described under 6.7/6.8. The size ranges determined are indicated in the graph.

**Fig. 4** Analysis of the B8 sample with the smallest concentration of DNA (<1 ng/ml as determined by the Threshhold^{®} assay) by the method of the invention as described under 6.7. The size ranges determined are indicated in the graph. No nucleic acids larger than 21 bp are detected.

**Fig. 5** Analysis of the 1 kb standard by the method of the invention as described under 6.7, with sample injection at 10 kV for 30 s.

**Fig. 6** Analysis of 10 bp standard (10 µg/ml starting concentration) without treatment (lower curve) and after betapropriolacton treatment for 16 hours (upper curve), injection at 9 kV for 5 s. The 1668 bp peak can still be detected at the correct size, even if in minimal concentration.

**Fig. 7** Calibration with the 1 kb standard and an internal standard (23 bases, ssDNA), identification of peaks according to mobility.

**Fig. 8** Comparison of the total amount of DNA in eight process steps from ten fermentations (DNA-Gehalt=DNA content (µg), Inprozesskontrollen= in-process controls).

## Claims

1. Method for analysing the presence and/or size distribution of nucleic acids, wherein a sample is separated by capillary gel electrophoresis, comprising
a) a hydrodynamic pre-injection of the capillary with water at 7 to 34 kPa for 2 to 10 s,
b) electrokinetic injection of the sample at 5-15 kV for 10-100 s
c) a hydrodynamic post-injection with water at 3 to 14 kPa for 2-10 s,
d) separation at 200-275 V/cm,
e) detection of nucleic acids.

2. Method of claim 1, wherein the hydrodynamic pre-injection is at 21 kPa for 5 s.

3. Method of claims 1 or 2, wherein the electrokinetic injection of the sample is at 10 kV for 90 s.

4. Method of claims 1 to 3, wherein the hydrodynamic post-injection is at 7 kPa for 5 s.

5. Method of any of the preceding claims, wherein the separation is at 200 to 250 V/cm, preferably at 250 V/cm.

6. Method of any of the preceding claims, wherein the detection is by laser-induced fluorescence.

7. Method of any of the preceding claims, wherein a washing step is carried out after steps a, b and/or c by contacting both ends of the capillary with water.

8. Method of any of the preceding claims, wherein a fluorescein solution is injected hydrodynamically at 7 to 34 kPa for 2 to 10 s between steps a and b.

9. Method of any of the preceding claims, wherein an internal standard for allocation of relative mobility values is injected between step a and b at 7-35 kPa for 1-10 s, preferably at 21 kPa for 5 s.

10. Method of any of the preceding claims, wherein the separation buffer comprises a dye suitable for detecting the nucleic acid, preferably an intercalating dye, most preferably EnhanCE dye.

11. Method of any of the preceding claims, wherein the nucleic acid is DNA, preferably genomic DNA and/or degradation products thereof.

12. Method of any of the preceding claims, wherein a sample to be analyzed for the presence of DNA is separated by capillary gel electrophoresis, comprising
a) a hydrodynamic pre-injection of the capillary with water at 21 kPa for 5 s,
b) electrokinetic injection of the sample at 10 kV for 90 s,
c) a hydrodynamic post-injection with water at 7 kPa for 5 s,
d) separation at 250 V/cm,
e) detection of nucleic acids by laser-induced fluorescence,
wherein the separation buffer comprises an intercalating dye, wherein an internal standard and a fluorescein solution are injected between step a and b at 7 to 35 kPa for 1-10 s, preferably at 21 kPa for 5 s, and wherein a washing step is carried out after the steps of injection of the internal standard, b and/or c.

13. Method of any of the preceding claims, wherein the sensitivity of the method is at least 20 pg/ml for a DNA fragment of 72 bp, preferably at least 9 pg/ml for a DNA fragment of 72 bp.

14. Method of any of the preceding claims, wherein the capillary is a fused silica capillary with a neutral inner coating, preferably a polacrylamide coating.

15. Method of any of the preceding claims, wherein the capillary is a fused silica capillary with an outer polyimid coating, wherein up to 2 mm at both ends of the capillary are not coated on the outer side.

16. Method of any of the preceding claims, wherein the capillary has an inner diameter of 100 µm.

17. Method of any of the preceding claims, wherein the capillary has a length of 40 cm to the detector and the separation is carried out for 20 to 40 min, preferably 23 to 35 min.

18. Method of any of the preceding claims, wherein the temperature of the system during separation is 17-25°C, preferably 20°C.

19. Method of any of the preceding claims, wherein the capillary is under a pressure of 14-69 kPa, preferably 34 kPa during separation.

20. Method of any of the preceding claims, wherein the separation buffer is a Tris-Borat buffer with a pH of 8 to 9.5, preferably a pH of 8.9.

21. Method of any of the preceding claims, wherein the separation is carried out with a PACE MDQ Molecular Characterization System.

22. Method of any of the preceding claims, wherein the sample is a pharmaceutical composition for therapy or vaccination and is to be analyzed for the presence of genomic DNA.

23. Method of any of the preceding claims, wherein the sample has been pretreated with a method comprising steps wherein
i) the sample is digested with Proteinase K in the presence of SDS,
ii) nucleic acids are extracted, and
iii) optionally, the nucleic acids are concentrated by centrifugation over a membrane with a cut-off size of 10 kDa.

24. Method for determining the size of a nucleic acid, comprising carrying out the method of any of the preceding claims, wherein the size of the nucleic acid is determined by comparison with an internal or external size standard.

25. Method for determining the size distribution of nucleic acids in a sample, comprising carrying out the method of any of the preceding claims, wherein the size distribution of the nucleic acids is determined by comparison with an internal or external size standard.

26. Method of claims 24 or 25, wherein the signal detected in step e is transformed into a curve in coordinates showing intensity versus time or mobility, and the curve is compared with the corresponding curve of the size standard for assigning a size to a nucleic acid.

27. Method for determining the size distribution of nucleic acids in a sample, comprising carrying out the method of claim 26, calculating the area under the curve for a size range of interest and comparing it with the total area under the curve, whereby the percentage of nucleic acids in the size range of interest is determined.

28. Use of the method of any of the preceding claims for quality control of a sample comprising proteins or nucleic acids for therapy or vaccination.

29. Use of the method of any of the preceding claims for the preparation of a vaccine, preferably a vaccine against influenza.

30. Method of analysing the presence and/or size distribution of nucleic acids in a sample from the preparation of a vaccine, comprising separating the sample by capillary gel electrophoresis and detecting nucleic acids by laser induced fluorescence.

31. Method of claim 30, wherein the nucleic acids are genomic DNA and/or degradation products thereof.

32. Method of claims 30 or 31, wherein the sample is from preparation of an influenza vaccine.

33. Method of claims 30 to 32, wherein the sample is analysed according to the method of claims 1 to 27.

34. Vaccine analysed by the method of any preceding claim.
